(19) Europäisches Patentamt
European Patent Office
Office européen des brevets

(11) Veröffentlichungsnummer: **0 264 802 B1**

(12) **EUROPÄISCHE PATENTSCHRIFT**

(45) Veröffentlichungstag der Patentschrift: **09.12.92**

(51) Int. Cl.5: **C07K 5/00**, C07K 7/00, C07C 269/00, C07C 271/00

(21) Anmeldenummer: **87114992.8**

(22) Anmeldetag: **14.10.87**

(54) **Synthese von Peptid-aminoalkylamiden und Peptid-hydraziden mittels Festphasenmethode.**

(30) Priorität: **21.10.86 DE 3635670**

(43) Veröffentlichungstag der Anmeldung: **27.04.88 Patentblatt 88/17**

(45) Bekanntmachung des Hinweises auf die Patenterteilung: **09.12.92 Patentblatt 92/50**

(84) Benannte Vertragsstaaten: **AT BE CH DE ES FR GB GR IT LI LU NL SE**

(56) Entgegenhaltungen:
**US-A- 4 108 846**

**JOURNAL OF THE CHEMICAL SOCIETY, CHEMICAL COMMUNICATIONS, 1982, Seiten 587-589, London, GB; R.C. SHEPPARD et al.: "A new protecting group combination for solid phase synthesis of protected peptides"**

**CHEMICAL ABSTRACTS, Band 98, Nr. 15, 11. April 1983, Seite 689, Zusammenfassung Nr. 126602q, Columbus, Ohio, US; R.C. SHEPPARD et al.: "Acid-labile resin linkage agents for use in solid phase peptide synthesis", & INT. J. PEPT. PROTEIN RES. 1982, 20(5), 451-4**

**TETRAHEDRON LETTERS, Band 28, Nr. 46, 1987, Seiten 5647-5650, Pergamon Journals Ltd, Oxford, GB; G. BREIPOHL et al.: "Solid phase synthesis of peptide aminoalkylamides development and application of new linking agents"**

(73) Patentinhaber: **HOECHST AKTIENGESELLSCHAFT**
**Postfach 80 03 20**
**W-6230 Frankfurt am Main 80(DE)**

(72) Erfinder: **Breipohl, Gerhard, Dr.**
**Geisenheimer Strasse 95**
**W-6000 Frankfurt am Main 71(DE)**
Erfinder: **Knolle, Jochen, Dr.**
**Höchster Strasse 21**
**W-6239 Kriftel(DE)**

EP 0 264 802 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Einführung eines Aminoalkylamids in das C-terminale Ende eines biologisch wirksamen Peptids hat sich in einigen Fällen positiv auf die metabolische Stabilität und die Wirksamkeit ausgewirkt. Bei der Herstellung der so modifizierten Peptide bediente man sich der klassischen Fragmentkupplung in Lösung (EP-A-179 332).

Bei der Festphasensynthese von Peptiden (vgl. Patchornik, Cohen in Perspectives in Peptide Chemistry, Seiten 118 - 128 (Karger, Basel 1981)) werden die reaktiven Ketten oft nicht direkt auf den Kunstharzkörper aufgepfropft, sondern durch sogenannte Spacer oder Links mit dem Trägermaterial verbunden. Aus der Literatur (z.B. Atherton, Sheppard in Perspectives in Peptide Chemistry, Seiten 101 - 117 (Karger, Basel 1981)) sind beispielsweise solche Spacer (sogenannte "linkage agents") bekannt, welche die Formeln VI, VII und VIII aufweisen.

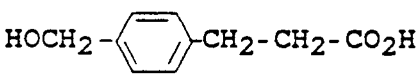

(VI)

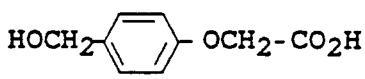

(VII)

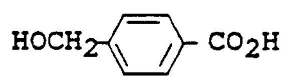

(VIII)

In CA 98: 126602 q werden säurelabile Spacer der Formel VIIa

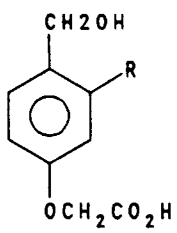

(R = H, OMe) (VIIa)

beschrieben. Mit Hilfe dieser Spacer lassen sich in der Festphase direkt nur Peptide, die C-terminal eine freie Carbonsäuregruppe aufweisen, synthetisieren. Nach Abspaltung des Peptides liegt der Spacer unverändert vor, das heißt es hat keine Spacerfragmentübertragung stattgefunden.

C-terminal durch Aminoalkylamid oder Hydrazid modifizierte Peptide sind mit Hilfe der in CA 98: 126602 q beschriebenen Spacer nach der Festphasenmethode nicht direkt, sondern nur durch einen kombinierten Mehrstufenprozeß, zugänglich, wobei an das nach in der Festphase hergestellte Peptid in Lösung ein Diaminoalkylrest angekuppelt werden müßte.

Aufgabe der Erfindung ist es Spacer bereitzustellen, die es ermöglichen direkt mittels Festphasensynthese C-terminal durch Aminoalkylamid oder Hydrazid modifizierte Peptide herzustellen. Diese Aufgabe wird durch die Verbindungen der Formel I gelöst.

(I)

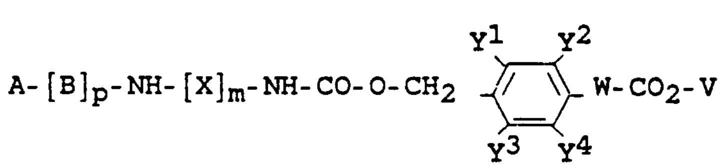

Die vorliegende Erfindung betrifft daher Verbindungen der Formel I in welcher

| | |
|---|---|
| A | Wasserstoff oder eine basenlabile oder gegen schwache Säuren labile Aminoschutzgruppe bedeutet, |
| B | für gleiche oder verschiedene Reste von Aminosäuren steht, |
| X | $(C_1-C_{12})$-Alkylen oder $(C_6-C_{10})$-Aryl-$(C_1-C_{12})$-alkylen bedeutet, |
| $Y^1$, $Y^2$, $Y^3$ und $Y^4$ | gleich oder verschieden sind und Wasserstoff, Methyl, Methoxy oder Nitro bedeuten, wobei mindestens einer dieser Reste Wasserstoff bedeutet, |
| V | Wasserstoff oder eine Carboxylschutzgruppe bedeutet, |
| W | $-[CH_2]_n-$ oder $-O-[CH_2]_n-$ bedeutet, |

m 0 oder 1 ist,
n eine ganze Zahl von 0 bis 6 ist und
p eine ganze Zahl von 0 bis 5 ist.

Es sind solche Verbindungen der Formel I bevorzugt, worin p = 0, 1 oder 2, insbesondere 0 ist und/oder worin m = 1 ist.

X ist vorzugsweise - $[CH_2]_q$-, wobei q = 1 - 12, vorzugsweise 1 - 8 sein kann.

Vorzugsweise mindestens 2, insbesondere mindestens 3 der Reste $Y^1$, $Y^2$, $Y^3$ und $Y^4$ bedeuten Wasserstoff.

Basenlabile oder gegen schwache Säuren labile Schutzgruppen sind insbesondere Urethanschutzgruppen, wie Fmoc, Ddz, Bpoc, Msc, Peoc, Pse und Tse, vorzugsweise Fmoc (siehe z.B. Hubbuch, Kontakte (Merck) 1979, Nr. 3, Seiten 14 - 23).

B steht für den Rest einer Aminosäure vorzugsweise einer α-Aminosäure, die, falls chiral, in der D- oder L-Form vorliegen kann. Bevorzugt sind Reste natürlich vorkommender Aminosäuren, deren Enantiomeren, Homologen, Derivate und einfache Metaboliten (vgl. z.B. Wünsch et al., Houben-Weyl 15/1 und 2, Stuttgart, Thieme 1974). So kommen beispielsweise in Frage,
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, Cyta, Daad, Dab, Dadd, Dap, Dapm, Dasu, Djen, Dpa, Dtc, Fel, Gln, Glu, Gly, Guv, hCys, His, hSer, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val sowie die Reste der entsprechenden enantiomeren D-Aminosäuren.

Funktionelle Gruppen in den Seitenketten der genannten Aminosäurereste können geschützt vorliegen. Geeignete Schutzgruppen sind bei Hubbuch, Kontakte (Merck) 1979, Nr. 3, Seiten 14 - 23 und beij Bullesbach, Kontakte (Merck) 1980, Nr. 1, Seiten 23 - 35 beschrieben. Bevorzugt sind solche Gruppen, die gegen Basen und schwache Säuren stabil sind und die mit Hilfe starker Säuren abgespaltet werden können.

Alkylen kann geradkettig oder verzweigt sein. Unter $(C_6-C_{10})$-Aryl wird beispielsweise Phenyl, Tolyl oder Naphthyl verstanden; bevorzugt ist Phenyl.

Eine Carboxylschutzgruppe V ist beispielsweise $(C_1-C_6)$-Alkyl oder $(C_7-C_{11})$-Aralkyl; bevorzugt ist Methyl, Ethyl, tert.Butyl, Benzyl und modifiziertes Benzyl, wie p-Chlor-, p-Brom-, p-Nitro- und p-Methoxybenzyl sowie das N-Analoge Picolyl, Im weiteren Sinne werden unter solchen Schutzgruppen aktivierte Estergruppen wie ONSu, OBt, OObt oder p-Nitrophenoxy verstanden.

Die Erfindung betrifft auch ein Verfahren zur Herstellung der Verbindungen der Formel I, das dadurch gekennzeichnet ist, daß man

a) eine Verbindung der Formel II

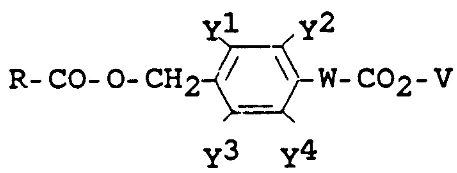

(II)

in welcher
R für eine nucleophil ablösbare Abgangsgruppe steht,
V für eine Carboxylschutzgruppe steht und
W, $Y^1$, $Y^2$, $Y^3$ und $Y^4$ wie in Anspruch 1 definiert sind, umsetzt mit einer Verbindung der Formel III

$A-[B]_p-NH-[X]_m-NH_2$ (III),

in welcher A für eine basenlabile oder gegen schwache Säuren labile Aminoschutzgruppe steht und B, X, p und m wie im Anspruch 1 definiert sind, und in der erhaltenen geschützten Verbindung der Formel I gegebenenfalls eine oder beide der Schutzgruppen A und/oder V unter Bildung der freien $NH_2$- und/oder $CO_2H$-Gruppe(n) abspaltet, wobei Verfahren bevorzugt sind, in denen selektiv V abgespalten wird, z.B. durch reduktive Spaltung mit Zn/Eisessig, oder

b) eine Verbindung der Formel I, in welcher A Wasserstoff bedeutet und B, X, $Y^1$, $Y^2$, $Y^3$, $Y^4$, V, W, m, n und p wie im Anspruch 1 definiert sind, umsetzt mit einer Verbindung der Formel IV

$A\text{-}[B]_{5\text{-}p}\text{-}OH$ (IV)

in welcher A, B und p wie oben definiert sind, A aber nicht Wasserstoff bedeutet, oder deren Aktivester, Halogenid oder Azid, und, falls V ≠ Wasserstoff ist, gegebenenfalls eine Carboxylschutzgruppe V abspaltet unter Bildung der Carboxylgruppe.

Eine nucleophil ablösbare Abgangsgruppe R ist beispielsweise Halogen, wie Chlor, Brom und Iod oder aktiviertes Aryloxy, wie p-Nitrophenoxy.

Die Umsetzung einer Verbindung der Formel II mit einer Verbindung der Formel III führt man vorzugsweise in einem aprotischen Lösungsmittel, wie z.B. THF, DMF, $CHCl_3$ oder $CH_2Cl_2$, vorteilhaft in Gegenwart einer Base, wie z.B. einem tertiären Amin, beispielsweise Ethyltriisopropylamin, Triethylamin oder Pyridin, wobei sich der Zusatz eines Acylierungskatalysators, wie z.B. DMAP, HOObt oder HOBt, vorteilhaft auswirkt, bei einer Temperatur zwischen 0 ° C und dem Siedepunkt des Reaktionsgemisches, bevorzugt zwischen O ° C und 40 ° C, durch.

Verbindungen der Formel I (A = Wasserstoff) setzt man um mit Verbindungen der Formel IV, deren Aktivester, Halogenid oder Azid vorzugsweise in einem organischen Lösungsmittel, wie DMF, vorteilhaft in Gegenwart einer Base, wie z.B. einem tert.Amin, bei einer Temperatur zwischen -10 ° C und dem Siedepunkt der Reaktionsmischung, bevorzugt bei Raumtemperatur. Geeignete Aktivester sind beispielsweise die ONSu-, OBt-, OObt- und p-Nitrophenoxy-Verbindungen. Bevorzugte Halogenderivate sind die Chloride. Zur Verbesserung der Löslichkeit kann Pyridiniumperchlorat zugesetzt werden

Verbindungen der Formel II stellt man beispielsweise her, indem man Ester der Formel IX

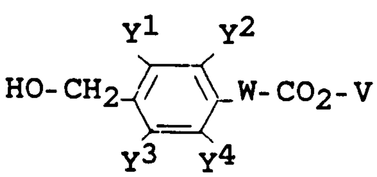

(IX)

worin $Y^1$, $Y^2$, $Y^3$, $Y^4$, W und V wie oben definiert sind, V aber nicht Wasserstoff bedeutet, umsetzt mit Phosgen oder Phosgenderivaten, wie z.B. Chlorameisensäurenitrophenylester, in einem aprotischen, polaren Lösungsmittel, z.B. THF oder DMF, im Gemisch mit einer tert. Base, beispielsweise einem tert. Amin wie Pyridin, bevorzugt im Verhältnis 1 : 1, bei einer Temperatur zwischen -40 ° C und Raumtemperatur, vorzugsweise zwischen - 20 ° C und 0 ° C.

Die Erfindung betrifft weiterhin die Verwendung einer Verbindung der Formel I in welcher V Wasserstoff bedeutet und A nicht Wasserstoff bedeutet, bei der Festphasensynthese von Verbindungen der Formel V

$P\text{-}NH\text{-}[X]_m\text{-}NH_2$ (V)

in welcher P für einen Peptidrest aus $q \leqq p + 1$ $\alpha$-Aminosäuren steht und X, m und p wie oben definiert sind sowie ein Verfahren zur Herstellung eines Peptids der Formel V, in welcher P, X, m und p wie oben definiert sind, durch Festphasensynthese, das dadurch gekennzeichnet ist, daß man eine Verbindung der Formel I, in welcher A nicht Wasserstoff bedeutet und V für Wasserstoff steht an ein Harz kuppelt, die Schutzgruppe A abspaltet, stufenweise q-p durch basenlabile oder gegen schwache Säuren labile Aminoschutzgruppen temporär geschützte $\alpha$-Aminosäuren gegebenenfalls in Form ihrer aktivierten Derivate ankuppelt und nach beendetem Aufbau das Peptid der Formel V durch Behandeln mit einer mittelstarken bis starken Säure von Harz freisetzt, wobei gleichzeitig oder durch geeignete Maßnahmen daran anschließend temporär eingeführte Seitenkettenschutzgruppen wieder abgespalten werden.

Falls zur Verhinderung von Nebenreaktionen oder für die Synthese spezieller Peptide erforderlich, sind die funktionellen Gruppen in der Seitenkette von Aminosäuren durch geeignete Schutzgruppen (siehe z.B. T.W. Greene, "Protective Groups in Organic Syntheses", New York, John Wiley & Sons, 1981) zusätzlich geschützt, wobei in erster Linie Arg(Tos), Arg(Mts), Arg(Mtr), Asp(OBzl), Asp(OBut), Cys(4-MeBzl), Cys-(Acm), Cys(SBut), Glu(OBzl), Glu(OBut), His(Tos), His(Fmoc), His(Dnp), His(Trt), Lys(Cl-2), Lys (Boc), Met-(O), Ser(Bzl), Ser(But), Thr(Bzl), Thr(But) eingesetzt werden.

Die als Trägermaterial verwendeten Harze sind kommerziell erhältlich. Bevorzugt sind BHA- und MBHA-Harze.

Die Abspaltung des Peptides der Formel V erfolgt dann durch Behandlung mit in der Peptidsynthese üblicherweise verwendeten mittelstarken bis starken Säuren (z.B. Trifluoressigsäure, HF) wobei die im

Spacer enthaltene Urethanschutzgruppe gespalten wird.

Als Kupplungsreagenz für die Verbindung der Formel I (V = H) und die weiteren Aminosäurederivate können alle möglichen in der Peptidsynthese verwendeten Aktivierungs-Reagenzien, siehe z.B. Houben-Weyl, Methoden der organischen Chemie, Band 15/2, verwendet werden, insbesondere aber Carbodiimide wie z.B. N,N'-Dicyclohexylcarbodiimid, N,N'Diisopropylcarbodiimid oder N-Ethyl-N'-(3-dimethylaminopropyl)-carbodiimid. Die Kupplung kann dabei direkt durch Addition von Aminosäurederivat mit dem Aktivierungsreagenz und gegebenenfalls einem die Racemisierung unterdrückenden Zusatz wie z.B. 1-Hydroxybenzotriazol (HOBt) (W. König, R. Geiger, Chem. Ber. 103, 708 (1970) oder 3-Hydroxy-4-oxo-3,4-dihydrobenzotriazin (HOObt) (W. König, R. Geiger, Chem. Ber. 103, 2054 (1970) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung der Verbindung der Formel I (V = H) und der Aminosäurederivate mit einem der obengenannten Aktivierungsreagenzien kann in Dimethylformamid oder Methylenchlorid oder einer Mischung aus beiden durchgeführt werden. Das aktivierte Aminosäurederivat wird üblicherweise in einem 1,5- bis 4-fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockung der α-Aminogruppe des Peptidharzes durchzuführen

Der erfolgreiche Ablauf der Kupplungsreaktion kann mittels der Ninhydrin-Reaktion, wie z.B. von E. Kaiser et al. Anal. Biochem. 34 595 (1970) beschrieben, überprüft werden. Die Synthese kann auch automatisiert z.B. mit einem Peptid-Synthesizer Modell 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Bei der Abspaltung der Peptidamide vom Harz mit Fluorwasserstoff und Trifluoressigsäure werden als Kationenfänger üblicherweise Substanzen, wie Phenol, Kresol, Thiokresol, Thioanisol, Anisol, Ethandithiol, Dimethylsulfid, Ethylmethylsulfid oder eine Mischung von zwei oder mehr dieser Hilfsmittel zugesetzt. Die Trifluoressigsäure kann dabei auch durch geeignete Lösungsmittel, wie z.B. Methylenchlorid verdünnt angewendet werden.

Verwendete Abkürzungen:

| | |
|---|---|
| Fmoc | 9-Fluorenylmethyloxycarbonyl |
| Ddz | α,α-Dimethyl-3,5-dimethoxybenzyloxycarbonyl |
| Bpoc | 2-[Biphenylyl-(4)]-propyl-(2)-oxycarbonyl |
| Msc | Methylsulfonylethyloxycarbonyl |
| Peoc | Pyridyl-ethyloxycarbonyl |
| Pse | Phenylsulfonyl-ethyloxycarbonyl |
| Tse | Tolylsulfonyl-ethyloxycarbonyl |
| HONSu | N-Hydroxy-succinimid |
| HOBt | 1-Hydroxybenzotriazol |
| HOObt | 3-Hydroxy-4-oxo-3,4-dihydrobenzotriazin |
| THF | Tetrahydrofuran |
| DMF | Dimethylformamid |
| DMAP | Dimethylaminopyridin |

Die nachstehenden Beispiele dienen zur Erläuterung der vorliegenden Erfindung, ohne daß diese darauf beschränkt wäre.

**Beispiel 1: 4-Hydroxymethylphenoxyessigsäuremethylester**

18,2 g 4-Hydroxymethylphenoxyessigsäure werden zusammen mit 17,1 ml N,N-Diisopropylethylamin in 50 ml DMF gelöst und dann zur gerührten Lösung 6,1 ml Methyljodid gegeben. Dabei erwärmt sich die Mischung leicht. Nach 3 h ist die Reaktion beendet. Das Lösungsmittel wird abgezogen. Der Rückstand wird in Ether aufgenommen und einmal mit 0,5 N Salzsäure extrahiert. Die wäßrige Phase wird noch dreimal mit Ether extrahiert, die vereinigten Etherphasen mit wäßriger Natriumhydrogencarbonat-Lösung gewaschen und eingeengt. Der Rückstand wird in Essigester gelöst und über eine kurze Säule mit Kieselgel filtriert. Das nach dem Einengen erhaltene schwach gelbliche Öl kristallisierte beim Stehenlassen.

NMR und Massenspektrum stimmen mit der angegebenen Struktur überein.

**Beispiel 2:**

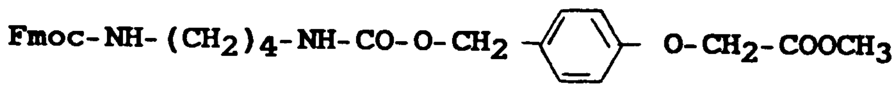

9,8, g 4-Hydroxymethylphenoxyessigsäuremethylester werden in 200 ml trockenem $CH_2Cl_2$ gelöst, dann 10,1 g Chlorameisensäure-p-nitrophenylester und 7 ml Trierthylamin zugegeben. Die Mischung wird ca. 6 h unter Rückfluß gekocht, bis das Edukt völlig umgesetzt ist. Dann wird eine Suspension von 15,5 g Fmoc-NH- $(CH_2)_4$-$NH_2$ (hergestellt durch Umsetzung von Boc-NH-$(CH_2)_4$-$NH_2$ mit Fmoc-ONSu und anschließender Boc-Abspaltung) in 100 ml trockenem $CH_2Cl_2$ sowie weitere 7 ml Triethylamin zugegeben und die Mischung unter Rückfluß gekocht. Nach beendeter Reaktion wird das Lösungsmittel abgezogen, der Rückstand mit Ether digeriert und abgesaugt. Der Filterrückstand wird mit wäßriger 1 N $Na_2CO_3$-Lösung und dann mit warmem Wasser gewaschen und im Exsikkator im Hochvakuum getrocknet.
Schmp. 122-124 ° C, NMR und Massenspektrum stimmen mit der angegebenen Struktur überein.

**Beispiel 3:**

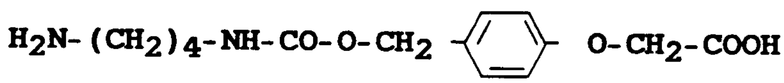

5,2 g des nach Beispiel 2 erhaltenen Esters werden in 100 ml Methanol suspendiert und 6 Äquivalente einer wäßrigen 1 N NaOH-Lösung zugegeben. beendeter Reaktion wird mit wäßriger 1 N HCl auf pH 3 gestellt und das Methanol abgezogen. Der Niederschlag wird abgesaugt, mit wenig $H_2O$ gewaschen und dann in Ether digeriert und erneut abgesaugt.
Schmp. ab 196 ° C (Zersetzung), NMR und Massenspektrum sind in Einklang mit der angegebenen Struktur.

**Beispiel 4:**

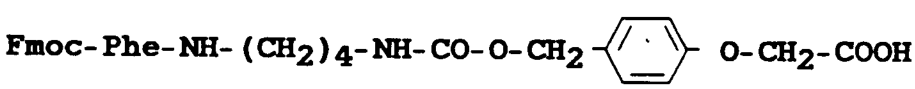

1,5 g des nach Beispiel 3 erhaltenen Produkts werden in 50 ml trockenem DMF suspendiert. Dann werden nacheinander 0,9 g Pyridiniumperchlorat (zur Verbesserung der Löslichkeit) sowie 2,6 g Fmoc-Phe-OObt und 0,5 ml Triethylamin zugegeben. Die Mischung wird bei Raumtemperatur gerührt. Nach beendeter Reaktion wird das Lösungsmittel abgezogen und der Rückstand zwischen Essigester und $H_2O$ verteilt. Die Wasserphase wird nochmals mit Essigester extrahiert und die vereinigten organischen Phasen getrocknet und eingeengt. Der Rückstand wird mit wenig $CHCl_3$ digeriert undf abgesaugt. Der Filterrückstand wird mit wenig Ether nachgewaschen und getrocknet.
Schmp. ab 140 ° C (Zersetzung), NMR und Massenspektrum sind in Übereinstimmung mit der angegebenen Struktur.

**Beispiel 5:**

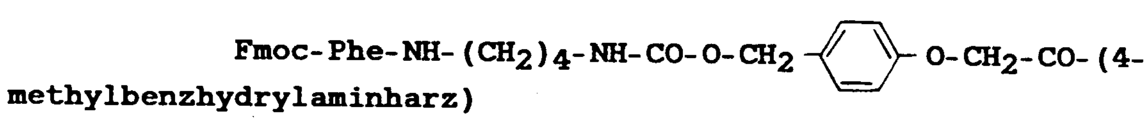

1,4 g der nach Beispiel 4 erhaltenen Fmoc-Phenylalaninspacersäure werden zusammen mit 350 mg HOBt in 40 ml trockenem DMF gelöst und zu 3,66 g 4-Methylbenzhydrylaminharz (Nova Biochem,

Beladung 0,4 mMol/g) gegeben. Anschließend versetzt man mit 0,6 ml Diisopropylcarbodiimid und läßt unter stetigem Durchmischen abreagieren. Nach beendeter Reaktion wird abgesaugt, mit DMF, Isopropanol, $CH_2Cl_2$ und tert.-Butylmethylether nachgewaschen und im Hochvakuum getrocknet. Beladung laut Elementaranalyse (N-Bestimmung) : 0,3 mMol/g.

**Beispiel 6: Synthese von [des-Tyr$^{24}$, des-Arg$^{23}$]-r-Atriopeptin III-(4-amino)butylamid**

Die Peptidsynthese erfolgt an 1 g des obigen Harzes unter Verwendung von Fmoc-Aminosäure-OOBt-Estern mit einem automatischen Peptidsynthesizer Modell 430A der Fa. Applied Biosystems und selbst modifizierten Syntheseprogrammen.

Dazu werden jeweils 1 mMol des entsprechenden Aminosäurederivates in die vom Hersteller gelieferten Cartridges eingewogen, Fmoc-Arg(Mtr)-OH, Fmoc-Asn-OH und Fmoc-Gln-OH werden zusammen mit 1,5 mMol HOBt in die Cartridges eingewogen. Die Voraktivierung dieser Aminosäuren erfolgt direkt in den Cartridges durch Lösen in 4 ml DMF und Zugabe von 2 ml einer 0,55 M Lösung von Diisopropylcarbodiimid in DMF. Die HOObt-Ester werden in 6 ml DMF gelöst und dann ebenso wie die in situ voraktivierten Aminosäuren Arginin Asparagin und Glutamin auf das vorher mit 20 % Piperidin in DMF entblockierte Harz gepumpt. Die in situ aktivierten Aminosäuren werden doppelt gekuppelt.

Nach beendeter Synthese wird das Peptid-butylamid unter gleichzeitiger Entfernung der Seitenkettenschutzgruppen mit Trifluoressigsäure, die Thioanisol und m-Kresol als Kationenfänger enthält, vom Harz abgespalten. Der nach Abziehen der Trifluoressigsäure erhaltene Rückstand wird mehrfach mit Essigester digeriert und zentrifugiert. Das verbliebene Rohpeptid wird zur Entfernung der Cysteinschutzgruppe mit Tributylphosphin in Trifluorethanol behandelt. Nach Entfernen des Lösungsmittels wird wiederum mit Essigester digeriert und zentrifugiert. Das reduzierte Rohpeptid wird sofort mid Jod in 80 %iger wäßriger Essigsäure oxidiert, der $I_2$-Überschuß mit Ascorbinsäure entfernt und die Reaktionsmischung nach Einengen auf ein kleines Volumen an ®Sephadex G25 mit wäßriger 1N Essigsäure entsalzt. Die das reine Peptid enthaltenden Fraktionen werden vereinigt und gefriergetrocknet.

Das Peptid entspricht laut Aminosäureanalyse in der Aminosäurezusammensetzung der angegebenen Formel.

**Beispiel 7: 4-Hydroxymethylphenoxyessigsäurephenacylester**

182 g 4-Hydroxymethylphenoxyessigsäure und 199 g α-Bromacetophenon werden in 600 ml trockenem DMF gelöst und dann bei 0°C 138 ml Triethylamin rasch zugetropft. Man läßt auf Raumtemperatur kommen und über Nacht rühren. Die DMF-Lösung wird auf 3, 5 l Wasser gegossen und die wäßrige Phase mit Ethylacetat extrahiert. Die organische Phase wird mit Wasser gewaschen, über Natriumsulfat getrocknet und eingeengt. Beim Eindampfen fällt das Produkt aus. Es wird abgesaugt, mit Ethylacetat/n-Hexan 1:1 gewaschen und im Hochvakuum getrocknet.
Schmp.: 94-95°C, NMR stimmt mit der angegebenen Struktur überein.

**Beispiel 8:**

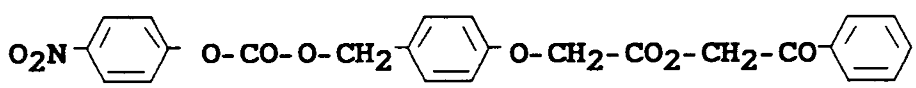

30 g 4-Hydroxymethylphenoxyessigsäurephenacylester werden unter Schutzgas in 500 ml einer Mischung aus THF/Pyridin 1:1 gelöst und auf - 20°C gekühlt. Anschließend werden 21 g Chlorameisensäure-p-nitrophenylester gelöst in 100 ml THF zugetropft. Nach 30 minütigem Rühren bei dieser Temperatur läßt man auf 0°C erwärmen und rührt die Mischung in 1 1 einer halbgestättigten wäßrigen NaCl-Lösung von 0°C ein und rührt 30 Minuten nach. Der Niederschlag wird abgesaugt, mit Eiswasser gewaschen und nach dem Trocknen mit n-Hexan verrieben.
Schmp.: 142-145°C, NMR ist in Übereinstimmung mit der angegebenen Struktur.

**Beispiel 9**

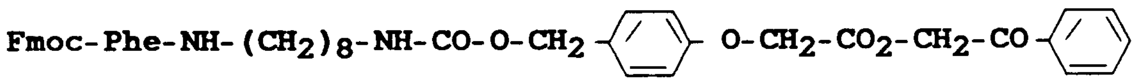

9,3 g der in Beispiel 8 hergestellten Verbindung, 12,25 g Fmoc-Phe-NH-$(CH_2)_8$-$NH_2$-trifluoracetat und 3,26 g HOObt werden als Festsubstanz in einen Kolben gegeben und dann mit einer Mischung von 2,58 g Ethyldiisopropylamin in 100 ml trockenem DMF übergossen. Die Mischung wird anschließend noch 3,5 Stunden bei 40 ° C gerührt und dann in 500 ml halbgesättigte wäßrige NaCl-Lösung eingerührt. Der ausgefallende Niederschlag wird abgesaugt, mit Eiswasser gewaschen und nach dem Trocknen mit Ether/Ethylacetat verrieben.
Schmp.: 147-150 ° C, NMR und MS stimmen mit der angegebenen Formel überein.

Folgende Verbindungen (Beispiele 10 bis 14) werden analog zu Beispiel 9 hergestellt:

**Beispiel 10:**

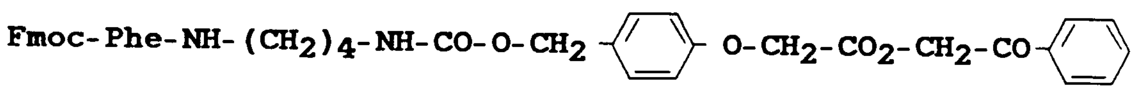

Schmp. 144-147 ° C, NMR und MS entsprechen der angegebenen Formel.

**Beispiel 11:**

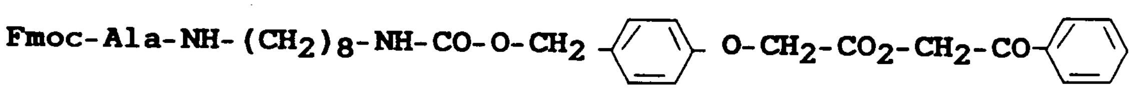

Schmp. 179-181 ° C, NMR und MS entsprechen der angegebenen Formel.

**Beispiel 12:**

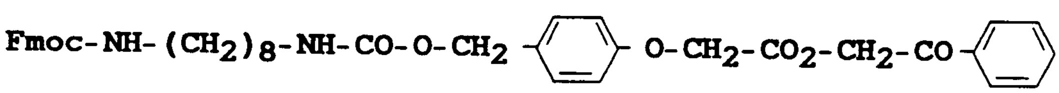

Schmp. 144-145 ° C, NMR und MS entsprechen der angegebenen Formel.

**Beispiel 13**

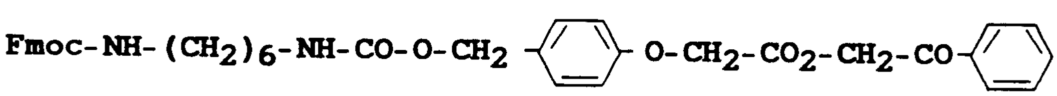

Schmp. 172-175 ° C, NMR entspricht der angegebenen Formel.

**Beispiel 14:**

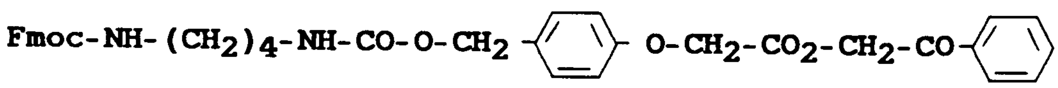

Schmp. 165-166 ° C, NMR entspricht der angegebenen Formel.

**Beispiel 15:**

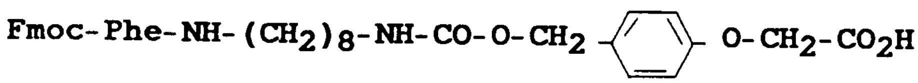

8,4 g 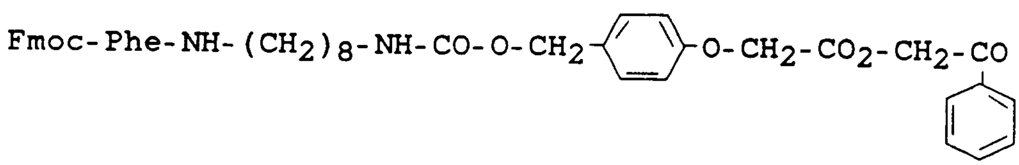

werden in einer Mischung aus 150 ml Eisessig und 50 ml Dichlormethan suspendiert und portionsweise mit 12 g Zinkpulver, das zuvor durch Waschen mit 1 N HCl und trockenem Ethanol aktiviert wurde, versetzt. Nach wenigen Minuten wird die Suspension unter leichter Wärmetönung dicker und schlecht rührbar. Deshalb werden weitere 80 ml Eisessig und 50 ml Dichlormethan zugesetzt und über Nacht weitergerührt. Danach wird übert einen Klärschichtfilter abgesaugt, mit Eisessig und Dichlormethan nachgewaschen. Das Filtrat wird eingeengt, das als Rückstand verbleibende Öl in wenig Dichlormethan aufgenommen und mit Ethylacetat und Ether verrührt. Das ausgefallene Produkt wird abgesaugt und im Hochvakuum getrocknet. Schmp.: ab 160 ° C Zersetzung, NMR und MS sind im Einklang mit der angegebenen Formel.

Nach der in Beispiel 15 beschriebenen Methode werden auch die Verbindungen der Beispiele 16 bis 18 hergestellt:

**Beispiel 16:**

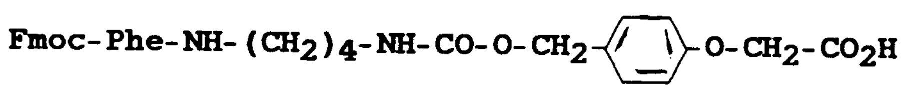

Schmp.: ab 150 ° C Zersetzung, NMR und MS im Einklang mit der angegebenen Formel.

**Beispiel 17:**

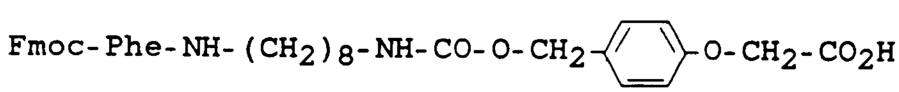

Schmp.: ab 160 ° C Zersetzung, NMR und MS im Einklang mit der angegebenen Formel.

**Beispiel 18:**

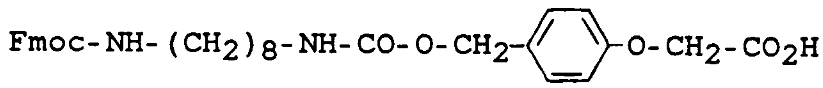

Schmp.: ab 154 ° C Zersetzung, NMR und MS im Einklang mit der angegebenen Formel.

**Beispiel 19:**

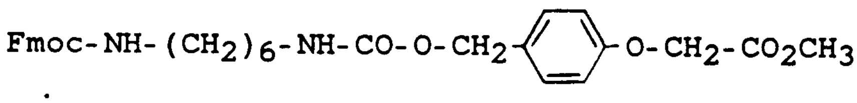

Die Synthese erfolgt analog Beispiel 2.
Schmp.: 115-118 ° C, NMR und MS stimmen mit der angegebenen Formel überein.

**Beispiel 20:**

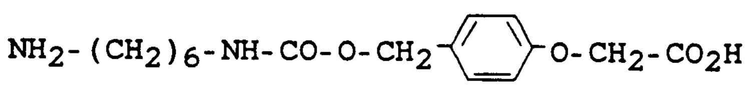

wurde nach der in Beispiel 3 beschriebenen Methode hergestellt.
Schmp.: 184-187 ° C Zersetzung, NMR und MS stimmen mit der angegebenen Formel überein.

**Beispiel 21:**

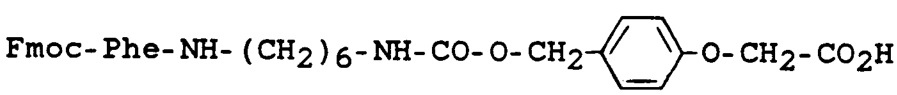

Die Synthese erfolgt analog Beispiel 4.
Schmp.: ab 120 ° C Zersetzung, NMR und MS stimmen mit der angegebenen Formel überein.

**Patentansprüche**
**Patentansprüche für folgende Vertragsstaaten : AT, BE, CH, DE, FR, GB, GR, IT, LI, LU, NL, SE**

**1.** Verbindung der Formel I

(I)

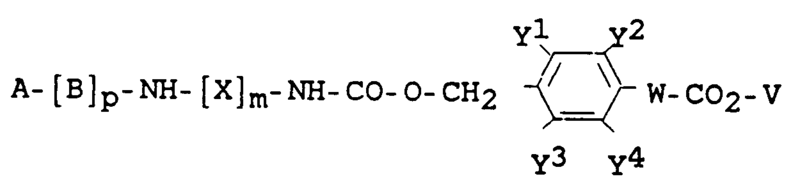

in welcher

| | |
|---|---|
| A | Wasserstoff oder eine basenlabile oder gegen schwache Säuren labile Aminoschutzgruppe bedeutet, |
| B | für gleiche oder verschiedene Reste von Aminosäuren steht, |

| | |
|---|---|
| X | $(C_1-C_{12})$-Alkylen oder $(C_6-C_{10})$-Aryl-$(C_1-C_{12})$-alkylen bedeutet, |
| $Y^1$, $Y^2$, $Y^3$ und $Y^4$ | gleich oder verschieden sind und Wasserstoff, Methyl, Methoxy oder Nitro bedeuten, wobei mindestens einer dieser Reste Wasserstoff bedeutet, |
| V | Wasserstoff oder eine Carboxylschutzgruppe bedeutet, |
| W | $-[CH_2]_n-$ oder $-O-[CH_2]_n-$ bedeutet, |
| m | 0 oder 1 ist, |
| n | eine ganze Zahl von 0 bis 6 ist und |
| p | eine ganze Zahl von 0 bis 5 ist. |

**2.** Verbindung der Formel I gemäß Anspruch 1, in welcher p = 0, 1 oder 2 ist.

**3.** Verbindung der Formel I gemäß Anspruch 1 oder 2, in welcher m = 1 ist.

**4.** Verbindung der Formel I gemäß einem der Ansprüche 1 - 3, in welcher X $-[CH_2]_q-$ und q eine ganze Zahl von 1 - 12 bedeutet.

**5.** Verbindung der Formel I gemäß einem der Ansprüche 1 - 4, in welcher mindestens 2 der Reste $Y^1$, $Y^2$, $Y^3$ und $Y^4$ Wasserstoff bedeuten.

**6.** Verfahren zur Herstellung einer Verbindung gemäß einem der Ansprüche 1 - 5, dadurch gekennzeichnet, daß man

a) eine Verbindung der Formel II

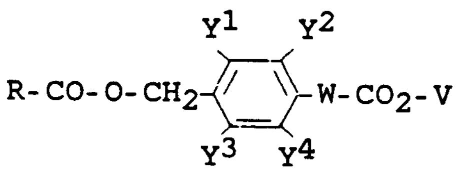

(II)

in welcher
R für eine nucleophil ablösbare Abgangsgruppe steht,
V für eine Carboxylschutzgruppe steht und
W, $Y^1$, $Y^2$, $Y^3$ und $Y^4$ wie im Anspruch 1 definiert sind, umsetzt mit einer Verbindung der Formel III

$A-[B]_p-NH-[X]_m-NH_2$ (III),

in welcher A für eine basenlabile oder gegen schwache Säuren labile Aminschutzgruppe steht und B, X, p und m wie im Anspruch 1 definiert sind, und in der erhaltenen geschützten Verbindung der Formel I gegebenenfalls eine oder beide der Schutzgruppen A und/oder V unter Bildung der freien $NH_2$- und/oder $CO_2H$-Gruppe(n) abspaltet oder

b) eine Verbindung der Formel I, in welcher A Wasserstoff bedeutet und B, X, $Y^1$, $Y^2$, $Y^3$, $Y^4$, V, W, m, n und p wie im Anspruch 1 definiert sind, umsetzt mit einer Verbindung der Formel IV

$A-[B]_{5-p}-OH$ (IV)

in welcher A, B un p wie oben definiert sind, A aber nicht Wasserstoff bedeutet, oder deren Aktivester, Halogenid oder Azid, und, falls V ≠ Wasserstoff ist, gegebenenfalls eine Carboxylschutzgruppe V abspaltet unter Bildung der Carboxylgruppe

**7.** Verwendung einer Verbindung der Formel I gemäß einem der Ansprüche 1 - 5, in welcher V Wasserstoff bedeutet und A nicht Wasserstoff bedeutet, bei der Festphasensynthese von Verbindungen der Formel V

$P-NH-[X]_m-NH_2$ (V)

in welcher P für einen Peptidrest aus $q \leqq p + l$ $\alpha$-Aminosäuren steht und X, m und p wie in Anspruch 1 definiert sind.

8. Verfahren zur Herstellung eines Peptids der Formel V, in welcher P, X, m und p wie Anspruch 7 definiert sind, durch Festphasensynthese, dadurch gekennzeichnet, daß man eine Verbindung der Formel I gemäß einem der Ansprüche 1 - 5, in welcher A nicht Wasserstoff bedeutet und V für Wasserstoff steht, an ein Harz kuppelt die Schutzgruppe A abspaltet, stufenweise q-p durch basenlabile oder gegen schwache Säuren labile Aminoschutzgruppen temporär geschützte α-Aminosäuren gegebenenfalls in Form ihrer aktivierten Derivate ankuppelt und nach beendetem Aufbau das Peptid der Formel V durch Behandeln mit einer mittelstarken bis starken Säure vom Harz freisetzt, wobei gleichzeitig oder durch geeignete Maßnahmen daran anschließend temporär eingeführte Seitenkettenschutzgruppen wieder abgespalten werden

**Patentansprüche für folgenden Vertragsstaat : ES**

1. Verfahren zur Herstellung einer Verbindung der Formel I

(I)

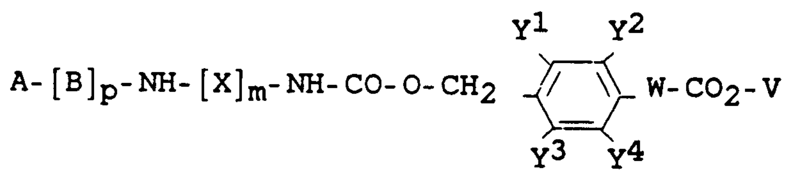

in welcher

| | |
|---|---|
| A | Wasserstoff oder eine basenlabile oder gegen schwache Säuren labile Aminoschutzgruppe bedeutet, |
| B | für gleiche oder vershiedene Reste von Aminosäuren steht, |
| X | $(C_1-C_{12})$-Alkylen oder $(C_6-C_{10})$-Aryl-$(C_1-C_{12})$-alkylen bedeutet, |
| $Y^1$, $Y^2$, $Y^3$ und $Y^4$ | gleich oder verschieden sind und Wasserstoff, Methyl, Methoxy oder Nitro bedeuten, wobei mindestens einer dieser Reste Wasserstoff bedeutet, |
| V | Wasserstoff oder eine Carboxylschutzgruppe bedeutet, $-[CH_2]_n-$ oder $-O-[CH_2]-_n-$ bedeutet, |
| m | 0 oder 1 ist, |
| n | eine ganze Zahl von 0 bis 6 ist und |
| p | eine ganze Zahl von 0 bis 5 ist, dadurch gekennzeichnet, daß man |

a) eine Verbindung der Formel II

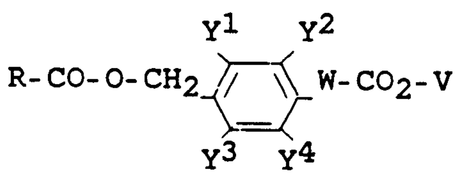

(II)

in welcher
R für eine nucleophil ablösbare Abgangsgruppe steht,
V für eine Carboxylschutzgruppe steht und
W, $Y^1$, $Y^2$, $Y^3$ und $Y^4$ wie oben definiert sind, umsetzt mit einer Verbindung der Formel III

$A-[B]_p-NH-[X]_m-NH_2$ (III)

in welcher A für eine basenlabile oder gegen schwache Säuren labile Aminoschutzgruppe steht und B, X, p und m wie oben definiert sind, und in der erhaltenen geschützten Verbindung der Formel I gegebenenfalls eine oder beide der Schutzgruppen A und/oder V unter Bildung der freien $NH_2$- und/oder $CO_2H$-Gruppe(n) abspaltet,
oder
b) eine Verbindung der Formel I, in welcher A Wasserstoff bedeutet un B, X, $Y^1$, $Y^2$, $Y^3$, $Y^4$, V, W, m, n und p wie oben definiert sind, umsetzt mit einer Verbindung der Formel IV

$A\text{-}[B]_{5\text{-}p}\text{-}OH$ (IV)

in welcher A, B und p wie oben definiert sind, A aber nicht Wasserstoff bedeutet, oder deren Aktivester, Halogenid oder Azid, und, falls V ≠ Wasserstoff ist, gegebenenfalls eine Carboxyxylschutzgruppe V abspaltet unter Bildung der Carboxylgruppe.

**2.** Verfahren zur Herstellung einer Verbindung der Formel I gemäß Anspruch 1, dadurch gekennzeichnet, daß p = 0, 1 oder 2 ist.

**3.** Verfahren zur Herstellung einer Verbindung der Formel I gemäß Anspruch 1 oder 2, dadurch gekennzeichnet, daß m = 1 ist.

**4.** Verfahren zur Herstellung einer Verbindung der Formel I gemäß einem der Ansprüche 1 - 3, dadurch gekennzeichnet, daß X $-[CH_2]_q-$ und q eine ganze Zahl von 1 - 12 bedeutet.

**5.** Verfahren zur Herstellung einer Verbindung der Formel I gemäß einem der Ansprüche 1 - 4, dadurch gekennzeichnet, daß mindestens 2 der Reste $Y^1$, $Y^2$, $Y^3$ und $Y^4$ Wasserstoff bedeuten.

**6.** Verfahren zur Herstellung eines Peptids der Formel V

$P\text{-}NH\text{-}[X]_m\text{-}NH_2$ (V)

in welcher P für einen Peptidrest aus q ≦ p + 1 α-Aminosäuren steht und X, m und p wie oben definiert sind, durch Festphasensynthese, das dadurch gekennzeichnet, ist, daß man eine Verbindung der Formel I, in welcher A nicht Wasserstoff bedeutet und V für Wasserstoff steht an ein Harz kuppelt, die Schutzgruppe A abspaltet, stufenweise q-p durch basenlabile oder gegen schwache Säuren labile Aminoschutzgruppen temporär geschützte α-Aminosäuren gegebenenfalls in Form ihrer aktivierten Derviate ankuppelt und nach beendetem Aufbau das Peptid der Formel V durch Bahandeln mit einer mittelstarken bis starken Säure von Harz freisetzt, wobei gleichzeitig oder durch geeignete Maßnahmen daran anschließend temporär eingeführte Seitenkettenschutzgruppen wieder abgespalten werden.

**Claims**

**Claims for the following Contracting States : AT, BE, CH, DE, FR, GB, GR, IT, LI, LU, NL, SE**

**1.** A compound of the formula I

(I)

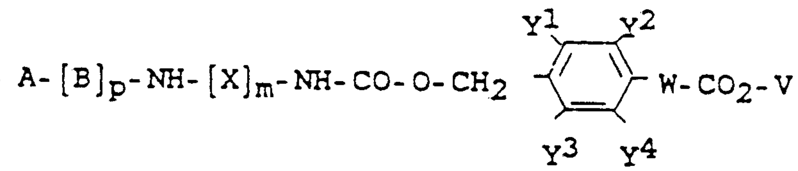

in which

| | |
|---|---|
| A | denotes hydrogen or an amino protective group which is labile to bases or labile to weak acids, |
| B | represents identical or different amino acid residues, |
| X | denotes $(C_1\text{-}C_{12})$-alkylene or $(C_6\text{-}C_{10})$-aryl-$(C_1\text{-}C_{12})$-alkylene, |
| $Y^1$, $Y^2$, $Y^3$ and $Y^4$ | are identical or different and denote hydrogen, methyl, methoxy or nitro, at least one of these radicals denoting hydrogen, |
| V | denotes hydrogen or a carboxyl protective group, |
| W | denotes $-[CH_2]_n-$ or $-O-[CH_2]_n-$, |
| m | is 0 or 1, |
| n | is an integer from 0 to 6, and |
| p | is an integer from 0 to 5. |

**2.** A compound of the formula I as claimed in claim 1, in which p is 0, 1 or 2.

**3.** A compound of the formula I as claimed in claim 1 or 2, in which m is 1.

**4.** A compound of the formula I as claimed in one of claims 1 - 3, in which X denotes $-[CH_2]_q-$, and q denotes an integer from 1 to 12.

**5.** A compound of the formula I as claimed in one of claims 1 - 4, in which at least 2 of the radicals $Y^1$, $Y^2$, $Y^3$ and $Y^4$ denote hydrogen.

**6.** A process for the preparation of a compound as claimed in one of claims 1 - 5, which comprises
a) reaction of a compound of the formula II

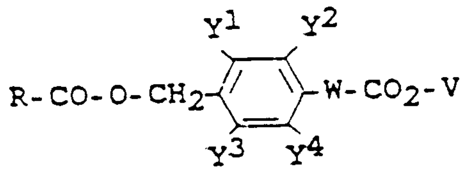

(II)

in which

R represents a leaving group which can be detached nucleophilically,
V represents a carboxyl protective group, and
W, $Y^1$, $Y^2$, $Y^3$ and $Y^4$ are as defined in claim 1, with a compound of the formula III

$A-[B]_p-NH-[X]_m-NH_2$ (III)

in which A represents an amino protective group which is labile to bases or labile to weak acids, and B, X, p and m are as defined in claim 1, and elimination of, where appropriate, one or both of the protective groups A and/or V in the resulting protected compound of the formula I, with the formation of the free $NH_2$ and/or $CO_2H$ group(s)
or
b) reaction of a compound of the formula I in which A denotes hydrogen, and B, X, $Y^1$, $Y^2$, $Y^3$, $Y^4$, V, W, m, n and p are as defined in claim 1, with a compound of the formula IV

$A-[B]_{5-p}-OH$ (IV)

in which A, B and p are as defined above, but A does not denote hydrogen, or its active ester, halide or azide, and, if V is not hydrogen, where appropriate elimination of a carboxyl protective group V with the formation of the carboxyl group.

**7.** The use of a compound of the formula I as claimed in one of claims 1 - 5, in which V denotes hydrogen, and A does not denote hydrogen, in the solid-phase synthesis of compounds of the formula V

$P-NH-[X]_m-NH_2$ (V)

in which P represents a peptide residue comprising $q \leqq p + 1$ $\alpha$-amino acids, and X, m and p are defined as in claim 1.

**8.** A process for the preparation of a peptide of the formula V, in which P, X, m and p are defined as in claim 7, by solid-phase synthesis, which comprises coupling a compound of the formula I, as claimed in one of claims 1-5, in which A does not denote hydrogen, and V represents hydrogen, to a resin, eliminating the protective group A, stepwise coupling on of q-p $\alpha$-amino acids which are, where appropriate, in the form of their activated derivatives and which have been temporarily protected by amino protective groups which are labile to bases or labile to weak acids and, after construction is complete, liberating the peptide of the formula V from the resin by treatment with a moderately strong to strong acid, the temporarily introduced side-chain protective groups being eliminated again at the same time or, by suitable measures, subsequent thereto.

**Claims for the following Contracting State : ES**

**1.** A process for the preparation of a compound of the formula I

(I) 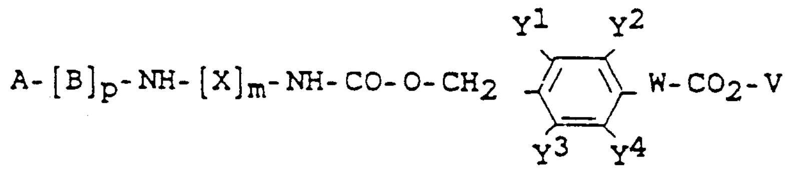

in which

| | |
|---|---|
| A | denotes hydrogen or an amino protective group which is labile to bases or labile to weak acids, |
| B | represents identical or different amino acid residues, |
| X | denotes $(C_1-C_{12})$-alkylene or $(C_6-C_{10})$-aryl-$(C_1-C_{12})$-alkylene, |
| $Y^1$, $Y^2$, $Y^3$ and $Y^4$ | are identical or different and denote hydrogen, methyl, methoxy or nitro, at least one of these radicals denoting hydrogen, |
| V | denotes hydrogen or a carboxyl protective group, |
| [lacuna] | denotes $-[CH_2]_n-$ or $-O-[CH_2]_n-$, |
| m | is 0 or 1, |
| n | is an integer from 0 to 6, and |
| p | is an integer from 0 to 5, which comprises |

a) reaction of a compound of the formula II

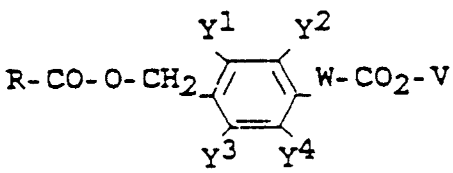 (II)

in which

R represents a leaving group which can be detached nucleophilically,
V represents a carboxyl protective group, and
W, $Y^1$, $Y^2$, $Y^3$ and $Y^4$ are as defined above, with a compound of the formula III

$A-[B]_p-NH-[X]_m-NH_2$ (III)

in which A represents an amino protective group which is labile to bases or labile to weak acids, and B, X, p and m are as defined above, and elimination of, where appropriate, one or both of the protective groups A and/or V in the resulting protected compound of the formula I, with the formation of the free $NH_2$ and/or $CO_2H$ group(s)

or

b) reaction of a compound of the formula I in which A denotes hydrogen, and B, X, $Y^1$, $Y^2$, $Y^3$, $Y^4$, V, W, m, n and p are as defined above, with a compound of the formula IV

$A-[B]_{5-p}-OH$ (IV)

in which A, B and p are as defined above, but A does not denote hydrogen, or its active ester, halide or azide, and, if V is not hydrogen, where appropriate elimination of a carboxyl protective group V with the formation of the carboxyl group.

**2.** The process for the preparation of a compound of the formula I as claimed in claim 1, wherein p is 0, 1 or 2.

**3.** The process for the preparation of a compound of the formula I as claimed in claim 1 or 2, wherein m

is 1.

**4.** The process for the preparation of a compound of the formula I as claimed in any of claims 1 to 3, wherein X is $-[CH_2]_q-$ and q is an integer from 1 to 12.

**5.** The process for the preparation of a compound of the formula I as claimed in any of claims 1 to 4, wherein at least 2 of the radicals $Y^1$, $Y^2$, $Y^3$ and $Y^4$ denote hydrogen.

**6.** A process for the preparation of a peptide of the formula V

$P-NH-[X]_m-NH_2$ (V)

in which P represents a peptide residue comprising $q \leqq p + 1$ α-amino acids, and X, m and p are defined as above, by solid-phase synthesis, which comprises coupling a compound of the formula I in which A does not denote hydrogen, and V represents hydrogen, to a resin, eliminating the protective group A, stepwise coupling on of q-p α-amino acids which are, where appropriate, in the form of their activated derivatives and which have been temporarily protected by amino protective groups which are labile to bases or labile to weak acids and, after construction is complete, liberating the peptide of the formula V from the resin by treatment with a moderately strong to strong acid, the temporarily introduced side-chain protective groups being eliminated again at the same time or, by suitable measures, subsequent thereto.

**Revendications**

**Revendications pour les Etats contractants suivants : AT, BE, CH, DE, FR, GB, GR, IT, LI, LU, NL, SE**

**1.** Composé de formule I

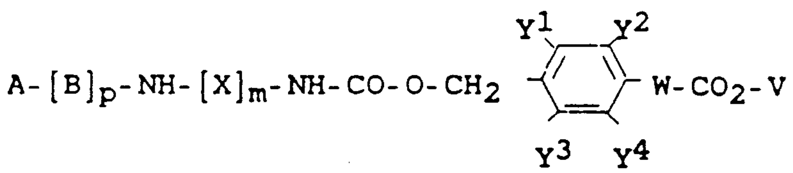

(I)

dans laquelle

| | |
|---|---|
| A | représente un atome d'hydrogène ou un groupe protecteur de fonction amino labile en présence d'acides faibles ou de bases, |
| B | représente des restes d'aminoacides identiques ou différents, |
| X | représente un radical alkylène en $C_1$-$C_{12}$ ou aryl($C_6$-$C_{10}$)-alkylène($C_1$-$C_{12}$), |
| $Y^1$, $Y^2$, $Y^3$ et $Y^4$ | sont identiques ou différents et représentent un atome d'hydrogène ou le groupe méthyle, méthoxy ou nitro, au moins un de ces radicaux représentant un atome d'hydrogène, |
| V | représente un atome d'hydrogène ou un groupe protecteur de fonction carboxy, |
| W | représente $-(CH_2)_n-$ ou $-O-(CH_2)_n-$, |
| m | est 0 ou 1, |
| n | est un nombre entier de 0 à 6 et |
| p | est un nombre entier de 0 à 5. |

**2.** Composé de formule I selon la revendication 1, dans lequel p est 0, 1 ou 2.

**3.** Composé de formule I selon la revendication 1 ou 2, dans lequel m est 1.

**4.** Composé de formule I selon l'une des revendications 1 à 3, dans lequel X représente $-[CH_2]_q-$ et q est un nombre entier de 1 à 12.

**5.** Composé de formule I selon l'une des revendications 1 à 4, dans lequel au moins deux des radicaux $Y^1$, $Y^2$, $Y^3$ et $Y^4$ représentent des atomes d'hydrogène.

**6.** Procédé pour la préparation d'un composé selon l'une des revendications 1 à 5, caractérisé en ce que a) on fait réagir un composé de formule II

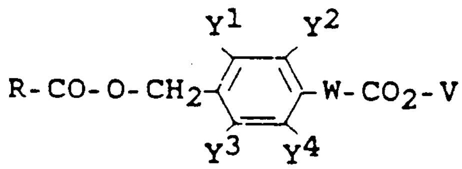

(II)

dans laquelle
R représente un groupe séparable pouvant être éliminé par substitution nucléophile,
V représente un groupe protecteur de fonction carboxy, et
W, $Y^1$, $Y^2$, $Y^3$ et $Y^4$ sont tels que définis dans la revendication 1,
avec un composé de formule III

$A\text{-}[B]_p\text{-}NH\text{-}[X]_m\text{-}NH_2$ (III)

dans laquelle A représente un groupe protecteur de fonction amino labile en présence d'acides faibles ou de bases, et B, X, p et m sont tels que définis dans la revendication 1, et, dans le composé de formule I protégé obtenu, éventuellement on élimine l'un des groupes protecteurs A ou V, ou les deux, avec formation du(des) groupe(s) $NH_2$ et/ou $CO_2H$ libre(s) ou
b) on fait réagir un composé de formule I, dans laquelle A représente un atome d'hydrogène et B, X, $Y^1$, $Y^2$, $Y^3$, $Y^4$, V, W, m, n et p sont tels que définis dans la revendication 1, avec un composé de formule IV

$A\text{-}[B]_{5\text{-}p}\text{-}OH$ (IV)

dans laquelle A, a et p sont tels que définis plus haut, mais A ne représente pas un atome d'hydrogène, ou un ester actif, halogénure ou azide de celui-ci, et, lorsque V est différent d'un atome d'hydrogène, éventuellement on élimine un groupe V protecteur de fonction carboxy, avec formation du groupe carboxy.

**7.** Utilisation d'un composé de formule I selon l'une des revendications 1 à 5, dans lequel V représente un atome d'hydrogène et A ne représente pas un atome d'hydrogène, dans la synthèse en phase solide de composés de formule V

$P\text{-}NH\text{-}(X)_m\text{-}NH_2$ (V)

dans laquelle P représente un reste peptidique constitué de $q \leqq p+1$ $\alpha$-aminoacides et X, m et p sont tels que définis dans la revendication 1.

**8.** Procédé pour la préparation d'un peptide de formule Y, dans lequel P, X, m et p sont tels que définis dans la revendication 7, par synthèse en phase solide, caractérisé en ce que l'on fixe par couplage à une résine un composé de formule I selon l'une des revendications 1 à 5, dans lequel A ne représente pas un atome d'hydrogène et V représente un atome d'hydrogène, on élimine le groupe protecteur A, on assemble graduellement par couplage q-p $\alpha$-aminoacides éventuellement sous forme de leurs dérivés activés, et temporairement protégés par des groupes protecteurs de fonction amino labiles en présence d'acides faibles ou de bases, et une fois terminée la synthèse, on sépare le peptide d'avec la résine par traitement par un acide moyennement fort à fort, en séparant en même temps, ou ensuite par des moyens appropriés, des groupes protecteurs de chaînes latérales, introduits temporairement.

**Revendications pour l'Etat contractant suivant : ES**

**1.** Procédé pour la préparation d'un composé de formule I

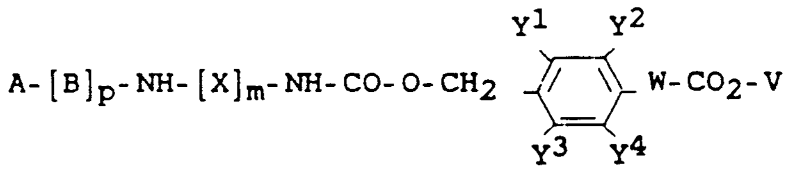 (I)

dans laquelle

A représente un atome d'hydrogène ou un groupe protecteur de fonction amino labile en présence d'acides faibles ou de bases,

B représente des restes d'aminoacides identiques ou différents,

X représente un radical alkylène en $C_1$-$C_{12}$ ou aryl($C_6$-$C_{10}$)-alkylène($C_1$-$C_{12}$),

$Y^1$, $Y^2$, $Y^3$ et $Y^4$ sont identiques ou différents et représentent un atome d'hydrogène ou le groupe méthyle, méthoxy ou nitro, au moins un de ces radicaux représentant un atome d'hydrogène,

V représente un atome d'hydrogène ou un groupe protecteur de fonction carboxy,

W représente $-(CH_2)_n-$ ou $-O-(CH_2)_n-$,

m est 0 ou 1,

n est un nombre entier de 0 à 6 et

p est un nombre entier de 0 à 5, caractérisé en ce que

a) on fait réagir un composé de formule II

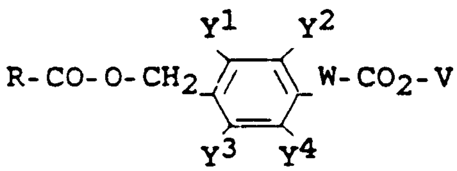 (II)

dans laquelle

R représente un groupe séparable pouvant être éliminé par substitution nucléophile,

V représente un groupe protecteur de fonction carboxy, et

W, $Y^1$ $Y^2$ $Y^3$ et $Y^4$ sont tels que définis plus haut, avec un composé de formule III

$A-[B]_p-NH-[X]_m-NH_2$ (III)

dans laquelle A représente un groupe protecteur de fonction amino labile en présence d'acides faibles ou de bases, et B, X, p et m sont tels que définis plus haut, et, dans le composé de formule I protégé obtenu, éventuellement on élimine l'un des groupes protecteurs A ou V, ou les deux, avec formation du(des) groupe(s) $NH_2$ et/ou $CO_2H$ libre(s) ou

b) on fait réagir un composé de formule I, dans laquelle A représente un atome d'hydrogène et B, X, $Y^1$, $Y^2$, $Y^3$, $Y^4$, V, W, m, n et p sont tels que définis plus haut, avec un composé de formule IV

$A-[B]_{5-p}-OH$ (IV)

dans laquelle A, B et p sont tels que définis plus haut, mais A ne représente pas un atome d'hydrogène, ou un ester actif, halogénure ou azide de celui-ci, et, lorsque V est différent d'un atome d'hydrogène, éventuellement on élimine un groupe V protecteur de fonction carboxy, avec formation du groupe carboxy.

**2.** Procédé pour la préparation d'un composé de formule I selon la revendication 1, caractérisé en ce que p est 0, 1 ou 2.

**3.** Procédé pour la préparation d'un composé de formule I selon la revendication 1 ou 2, caractérisé en ce que m est 1.

**4.** Procédé pour la préparation d'un composé de formule I selon l'une des revendications 1 à 3,

caractérisé en ce que X représente $-[CH_2]_q-$ et q est un nombre entier allant de 1 à 12.

5. Procédé pour la préparation d'un composé de formule I selon l'une des revendications 1 à 4, caractérisé en ce qu'au moins deux des radicaux $Y^1$, $Y^2$, $Y^3$ et $Y^4$ représentent des atomes d'hydrogène.

6. Procédé pour la préparation d'un peptide de formule V

$P-NH-(X)_m-NH_2$ (V)

dans laquelle P représente un reste peptidique constitué de $q \leqq p+1$ $\alpha$-aminoacides et X, m et p sont tels que définis plus haut, par synthèse en phase solide, caractérisé en ce que l'on fixe par couplage à une résine un composé de formule I dans lequel A ne représente pas un atome d'hydrogène et V représente un atome d'hydrogène, on élimine le groupe protecteur A, on assemble graduellement par couplage q-p $\alpha$-aminoacides éventuellement sous forme de leurs dérivés activés, et temporairement protégés par des groupes protecteurs de fonction amino labiles en présence d'acides faibles ou de bases, et une fois terminée la synthèse, on sépare le peptide d'avec la résine par traitement par un acide moyennement fort à fort, en séparant en même temps, ou ensuite par des moyens appropriés, des groupes protecteurs de chaînes latérales, introduits temporairement.